# EUROPEAN PATENT APPLICATION

(11) **EP 4 628 226 A1**
(43) Date of publication of application: **08.10.2025**
(21) Application number: 24168288.9
(22) Date of filing: 03.04.2024
(51) Int. Cl.: B06B 1/06, A61B 8/00

(54) **ULTRASONIC SENSOR SUBSTRATE, ULTRASONIC SENSOR DEVICE AND METHOD OF USE**

(71) Applicant: Amor Senoussi, Karim, 20 000 Casablanca (MA)
(72) Inventor: Amor Senoussi, Karim, 20 000 Casablanca (MA)
(74) Representative: Novagraaf International SA

(57) **Abstract**

An ultrasonic sensor substrate (100), made of a piezoelectric material and including:
- two opposite main surfaces (110, 120) each having a large surface area,
- two opposite side surfaces (130, 140) each having a small surface area and each adapted to receive a respective first electrode (230, 240),
wherein one of the main surfaces (120) is adapted to receive a second electrode and the other main surface (110) of the main surfaces includes at least two cavities (180).

## Description

### TECHNICAL FIELD

The present disclosure relates to an ultrasonic sensor, a device including the ultrasonic sensor and a method using the ultrasonic sensor device.

### BACKGROUND

Ultrasonic sensors have been used in various applications, for example in the field of medical devices in order to detect physiological parameters. Document US20160374599 discloses a sensor for non-invasive blood glucose monitoring.

### SUMMARY OF THE DISCLOSURE

An aspect of the present disclosure relates to an ultrasonic sensor substrate, made of a piezoelectric material and including:
- two opposite main surfaces each having a large surface area,
- two opposite side surfaces each having a small surface area and each adapted to receive a respective first electrode,
wherein one of the main surfaces is adapted to receive a second electrode and the other main surface of the main surfaces includes at least two cavities.

Such an ultrasonic sensor substrate may be used to detect physiological parameters and/or to analyse materials, such as smooth materials, gels or liquids. The cavities allow the flesh, skin or material to be analysed to penetrate the sensor and for the ultrasonic wave to efficiently travel through the flesh, skin or material. The substrate may generate ultrasonic volume waves in the longitudinal direction, i.e. between the two opposite side surfaces. The cavities are preferably bore holes.

Such a substrate thus allows to make an autonomous sensor performing both the emission and the reception functions. Preferably, the substrate is used without any other sensing element such as without an additional ultrasonic substrate, a specific receptor, a specific emitter, a light source, a laser, a CCD, etc.

Advantageously, the two cavities are arranged along a transversal axis of the ultrasonic sensor substrate. Such an arrangement may allow to increase the sensitivity of a corresponding sensor device.

Advantageously, at least another cavity is included, preferably arranged along the longitudinal axis of the ultrasonic sensor substrate with regard to the two cavities. For example, two other cavities may be added and arranged the longitudinal axis. The four cavities may be arranged crosswise. Again, such additional cavities may allow to increase the sensitivity of a sensor device using the ultrasonic sensor substrate. A maximum of ten, twelve of fourteen cavities may be present in the ultrasonic sensor substrate, depending on the size of the substrate. Preferably, cavities are regularly arranged, i.e. along a geometrical arrangement such as crosswise, square wise or in a shape of a circle.

Advantageously, the cavities have a height of 0.5 to 5 mm and a width or diameter of 1 to 5 mm. Such dimensions are particularly adapted for ultrasonic analysis and may increase the reliability of the corresponding sensor. Preferably, the cavities have a height of 0.5 to 2.8 mm, for example 0.8 to 1.8 mm and preferably 1 mm or 1.2 mm. The width or diameter of the cavities is preferably of 1.2 to 2.8 mm, preferably 1.5 or 1.6 mm. All the cavities may have the same shape and/or dimensions.

Preferably, the ultrasonic sensor substrate has a thickness (along the height of the cavities) larger than the height of the cavities and for example of 0.9 mm to 5.9 mm, preferably 1.1 mm to 4.1 mm, 1.6 mm to 2.8 mm and at most preferably 1.9 or 2.0 mm. The length of the ultrasonic sensor substrate, for example along the longitudinal axis, may be 3.8 to 12 mm, preferably 4.5 mm to 10.5 mm. The width of the ultrasonic sensor, for example along the transversal axis, may be two thirds or half of the length of the ultrasonic sensor.

Advantageously, the main surfaces and the side surfaces are flat and/or the side surfaces are orthogonal to at least one of the main surfaces. The substrate may thus have a global shape of a parallelepiped such as a rectangle parallelepiped, which is easy to manufacture and well adapted for a flat body part or a flat material sample to be analysed.

Advantageously, at least one of the main surfaces is curved and the side surfaces are orthogonal to an extremal tangent of the curved main surface. In the case the curved main surface is the other main surface provided with the cavities, the corresponding substrate is specifically adapted to be placed on a curved body part or a curved material sample to be analysed. For example, both main surfaces are curved and parallel, i.e. with a same curvature radius. Again preferably, the curvature radius is constant.

Advantageously, the piezoelectric material is at least one of PVDF-TrFE, PVDF-graphene and quartz, and is preferably PVDF-TrFE. Such materials allow to generate appropriate ultrasonic waves and recover ultrasonic signal(s) (i.e. detection signal) with a great sensitivity.

Advantageously, the other main surface comprises at least one pair of interdigitated electrodes and for example two pairs or more. Interdigitated electrodes contribute to measuring other physiological parameters.

Another aspect of the present disclosure is an ultrasonic sensor device including:
- the ultrasonic sensor substrate as described above,
- at least one first electrode on each side surface, and
- a second electrode on the one main surface.

Such an ultrasonic sensor device may measure physiological parameters with a great accuracy while being manufactured as a light and small device, since no other sensing element is required for the ultrasonic analysis.

Preferably, the second electrode may cover at least 50% of the area of the one main surface, preferably at least 75% and again preferably at least 90% of the area of the main surface. Such an ultrasonic sensor device allows to perform a non-invasive measurement of at least one physiological parameter on a human or animal being. It may also allow other measurements on smooth materials, gels or liquids.

Preferably, the ultrasonic sensor device includes a current generator connected to the first electrodes and configured to generate an electrical signal. The electrical signal may be of 0.001 MHz to 1 MHz and may be continuous and/or periodical, for example sinusoidal. For example, the electrical signal has a short duration such as less than 100 ms and preferably 2 ms to 45 ms, more preferably 6 ms to 32 ms.

Advantageously, the ultrasonic sensor device includes a detection device connected to one of the first electrodes and to the second electrode. For example, the detection device includes an analogic/digital converter and/or a microcontroller. The microcontroller may be configured to detect and/or analysed the detection signal obtained between the one first electrode and the second electrode.

Advantageously, the ultrasonic sensor device includes fixing means adapted to fix the ultrasonic sensor on a body part so that the other of the main surfaces applies pressure on the body part. The pressure may be enough for the skin and/or flesh to penetrate the cavities, and for example 0.01 N to 0.5 N. Alternatively, the ultrasonic sensor device comprises holding means and/or mechanical means, to be placed and hold on a body part or a material sample to be analysed.

Another aspect of the present disclosure relates to a method to measure at least one physiological parameter with the ultrasonic sensor device according to the aspect above, comprising the steps of:
- Generating an electrical signal between the first electrodes,
- Detecting a detection signal between one of the first electrodes and the second electrode,
- Computing a value of a physiological parameter from the detection signal.

Such a method allows a non-invasive measurement of a physiological parameter with a high accuracy and a limited cost. In particular, this method is self-sufficient and does not require any other technical means such as a second ultrasonic substrate, a CCD, a light source, a laser, etc.

Advantageously, the electrical signal is continuous and periodic. For example, the electrical signal corresponds to few periods, such as 2 to 10 periods.

Advantageously, the electrical signal is alternative and preferably sinusoidal or square wave.

Advantageously, the electrical signal has a frequency of more than 1 kHz and less than 1 MHz, for example 200 kHz or 700 kHz.

Another aspect of the present disclosure relates to a computer program configured to perform the above method steps. For example, the computer program is performed by a microcontroller or a microprocessor integrated in the ultrasonic sensor device or distant from the ultrasonic sensor device.

Another aspect of the present disclosure relates to a method to measure a physical parameter of a material sample such as a liquid, a gel or a solid material with the ultrasonic sensor device according to the aspect above, comprising the steps of:
- Generating an electrical signal between the first electrodes,
- Detecting a detection signal between one of the first electrodes and the second electrode,
- Computing a value of a physical parameter from the detection signal.

Another aspect of the present disclosure relates to a computer program configured to perform the above method steps.

### BRIEF DESCRIPTION OF THE DRAWINGS

Further advantages and preferred embodiments of the present disclosure will become apparent from the following detailed description and drawings, in which:
Figure 1 shows a three-quarter view of an ultrasonic sensor substrate according to an example of the present disclosure.
Figures 2A-2D show side views of alternative ultrasonic sensor substrates according to alternative examples of the present disclosure.
Figure 3 shows a schematic view of an ultrasonic sensor device including the ultrasonic sensor substrate of Figure 1.
Figure 4 shows a front view of an ultrasonic sensor substrate according to another example of the present disclosure.
Figure 5A shows blood sugars values obtained with an ultrasonic sensor device of the present disclosure (Y-axis) with regard to values obtained by medical analyses (X-axis). Figure 5B shows blood sugar values measured with a glucometer from a city pharmacy (Y-axis) with regard to values obtained by medical analyses (X-axis).

### DETAILED DESCRIPTION

Ultrasonic sensors are devices that utilize the principles of sound waves beyond the range of human hearing to detect and measure distances or objects. These sensors typically operate at frequencies above 20,000 hertz, which is the upper limit of human audibility.

One application of ultrasonic sensors is in proximity sensing and obstacle detection. The basic concept involves emitting ultrasonic pulses from a transducer and measuring the time it takes for the echoes to return after bouncing off an object. By calculating this time delay, the sensor can determine the distance to the object with high precision. These sensors are widely used in robotics, automotive parking systems, and industrial automation.

In robotics, ultrasonic sensors help robots navigate and avoid obstacles by providing real-time distance information. In parking systems, they assist drivers by emitting signals when the vehicle is too close to an object. The non-contact nature of ultrasonic sensing makes it suitable for applications where traditional contact sensors may be impractical or ineffective.

Moreover, ultrasonic sensors find applications in the medical field, such as in ultrasound imaging and diagnostics. In echography for example, ultrasonic waves are transmitted into the body, and the echoes are used to create detailed images of internal organs.

Piezoelectric materials are a class of materials that exhibit the piezoelectric effect, a phenomenon where they generate an electric charge in response to mechanical stress or deformation. Conversely, when an electric field is applied to these materials, they undergo a mechanical deformation.

Piezoelectric materials may be applied to sensors and actuators. When incorporated into sensors, such as pressure sensors, accelerometers, or vibration sensors, piezoelectric materials can convert mechanical signals into electrical signals, providing a reliable means of measuring various physical quantities. In the realm of actuators, these materials are employed to convert electrical energy into mechanical motion, enabling precise control in devices like inkjet printers and ultrasonic motors.

In addition to their use in sensors and actuators, piezoelectric materials find applications in energy harvesting. The ability of these materials to convert mechanical vibrations into electrical energy is harnessed to power small electronic devices and sensors in environments where traditional power sources may be impractical.

Piezoelectric materials may play a role in medical imaging technologies, particularly in ultrasound transducers. When an electric field is applied to a piezoelectric crystal, it vibrates and generates ultrasonic waves, which may be used for imaging internal structures in the human body (such as in echography).

The present disclosure relates to an ultrasonic sensor device including a substrate made of piezoelectric material. This ultrasonic sensor device may be used to perform measurements in the field of material science and medicine and will be described below in its use for the detection of at least one physiological parameter of a living organism, such as an animal and for example a mammal or a human being.

### Substrate (First Embodiment)

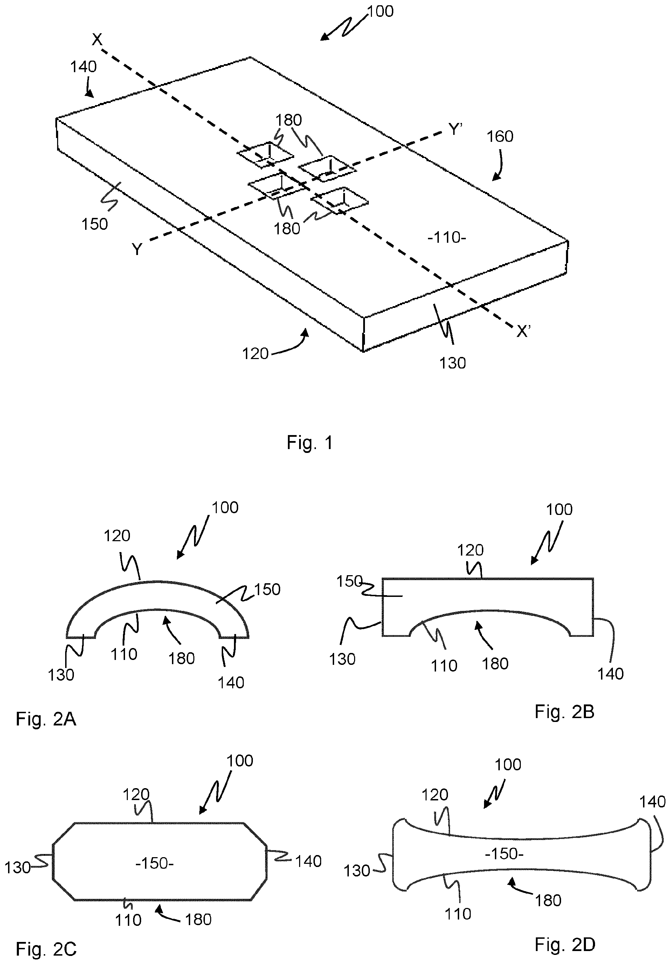

Now referring to Fig. 1, a substrate 100 may have the general form of polyhedron, for example a rectangular parallelepiped including two opposite main surfaces 110, 120, two opposite side surfaces 130, 140 and two opposite longitudinal surfaces 150, 160. In the specific example of Fig. 1, each surface is parallel to the opposite surface and the angles are orthogonal. The edges of the substrate 100 may be curved.

The main surface 110 visible in Fig. 1 comprises four cavities 180, all identical and disposed in a crosswise arrangement. In particular, two cavities may be aligned on the transversal axis Y-Y' of the substrate 100 and two other cavities may be aligned on the longitudinal axis X-X' of the substrate. The cavities are preferably centred on the main surface 110, for example arranged on or around a centre or barycentre of the main surface 110 and/or of the substrate 100. The cavities are bore holes, i.e. each with a single opening and an opposite wall inside the substrate 100.

Other shapes of the substrate 100 are visible in Figs. 2A-2D. Fig. 2A shows an example of a substrate 100 curved on the longitudinal axis X-X'. The curvature radius of each main surface 110, 120 is preferably constant and equal or substantially equal one to the other. The main surfaces 110, 120 may thus be parallel or substantially parallel one to the other. The side surfaces 130, 140 may be aligned on the same plan or be substantially parallel one to the other. A limited angle of each side surface 130, 140 with a common parallel plan may be acceptable, for example up to 5°.

Fig. 2B shows an example of a substrate 100 differing from that of Fig. 1 by having a curved main surface 110. The opposite main surface 120 may be flat. Such an embodiment may be advantageous to fit the shape of a body part, for example. Fig. 2C shows an example of a substrate 100 differing from that of Fig. 1 by having bevelled edges. This may be advantageous in order to simplify manufacturing and/or simplify the integration of the substrate in a sensor device. Fig. 2D shows an example of a substrate 100 different from that of Fig. 2B by having concave main surfaces 110, 120. A main surface 120 having a greater area, for example concave or convex, may be advantageous to collect a higher detection signal.

However, the substrate 100 is not limited to these exemplary forms and may result from any combination of these forms. The substrate 100 may generally include any form or geometry having at least two opposite side surfaces and two opposite main surfaces. Each surface and especially the main surfaces may be convex, concave, flat, bevelled or a combination of that. One of the main surfaces 110 has at least two cavities 180.

The dimensions of the substrate 100 are preferably smaller than a wavelength of the ultrasonic signal intended to be produced, for example twice smaller. For example, the greatest dimension of the substrate 100, i.e. the length of the substrate 100 measured along the longitudinal axis X-X', may thus be at most 12 mm. The substrate may also be long enough to include the cavities with sufficient material, and the length may be at least 3.8 mm. The substrate length may be preferably 4.5 mm to 10.5 mm.

The height, i.e. the thickness of the substrate 100 is preferably small with regard to the length, for example one third or one tenth. The substrate height may be 0.9 mm to 5.9 mm, preferably 1.1 mm to 4.1 mm, 1.6 mm to 2.8 mm and at most preferably 1.9 or 2.0 mm.

The width, i.e. the second greatest dimension of the substrate 100, for example measured along a transversal axis Y-Y', may be smaller than the length, for example two thirds, or half the length of the substrate 100. The width of the substrate 100 may be the width of one of the main surfaces 110, 120 and the length of one of the side surfaces 130, 140.

The cavities 180 may be square as shown in Fig. 1 but the general form is not limited: the cavities may be rectangular, circular, oval or have a more complex shape such as hexagonal. The cavity back wall, i.e. the wall parallel to the longitudinal axis X-X' (in the X-Y plane) may be flat or curved. The cavities preferably have a dimension smaller than the wavelength of the ultrasonic signal intended to be produced. For example, a dimension such as a width or a diameter of the cavities (i.e. along the axis X-X' and/or Y-Y') may be 1.2 to 2.8 mm, preferably 1.5 or 1.6 mm. The height of the cavities, i.e. along the thickness of the substrate 100, may be 0.5 to 2.8 mm, preferably 0.8 to 1.8 mm and more preferably 1.0 to 1.2 mm.

The cavities may be adapted according to the intended use of the sensor and their dimensions may be adapted according to the wavelength of the ultrasonic wave intended to be produced. Preferably, the cavities have all the same dimensions. At least two cavities are provided, and possibly four, six or eight cavities. The number of cavities may thus be even. Up to fourteen cavities may be provided in the substrate.

The substrate 100 is produced from a piezoelectric material adapted to produce ultrasonic waves. The piezoelectric material can then be chosen among quartz, barium titanate, lead zirconate titanate (PZT) and polyvinylidene fluoride (PVDF) and PVDF copolymers or terpolymers such as PVDF-TriFluoroEthylene, P(VDF-TrFE); or PvDF-graphene or PVDF-TriFluoroEthylene-ChlorotriFluoroEthylene, P(VDF-TrFE-CFE). The substrate may be produced by moulding, machining and/or 3D printing. Preferably, the substrate 100 is made of PVDF-TrFE.

### Sensor Device

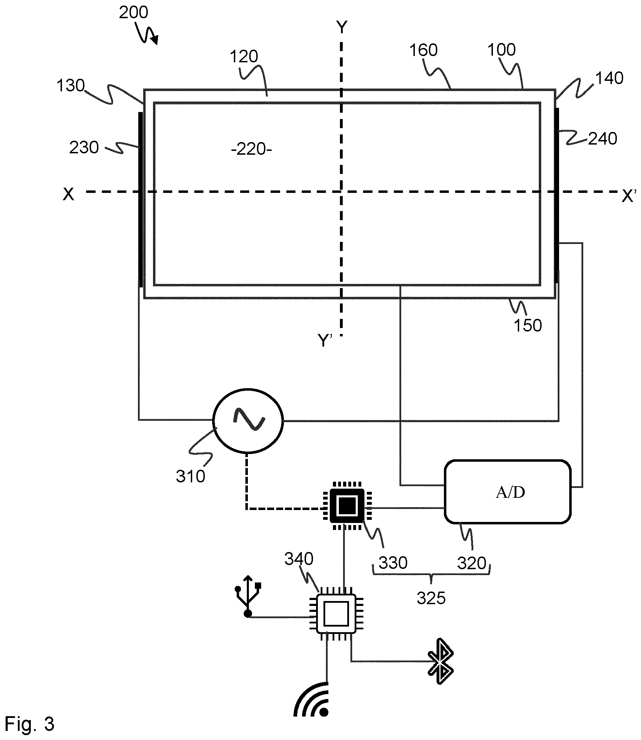

Fig. 3 shows a sensor device 200 including a substrate 100 with at least three electrodes. At least two first electrodes 230, 240 are each mounted or fixed on a respective side surface 130, 140, and in electrical contact with the substrate 100. At least a second electrode 220 is mounted or fixed on the main surface 120 opposite to that having the cavities 180 (not shown in Fig. 3) and in electrical contact with the substrate 100. The electrodes may be in any conductive material, for example copper, gold, aluminium and their alloys and combinations.

The first electrodes 230, 240 are electrically connected to a current generator 310 and one of the first electrodes 230, 240 and the second electrode 220 are connected to a signal detector or detection device 325 formed for example by an analogic/digital converter 320 and a microprocessor or microcontroller 330. The microcontroller 330 may also control the current generator 310, for example through a digital/analogic converter, or the current generator 310 may be controlled by a different microcontroller (not shown).

A general microprocessor 340 may control the microcontroller 330 and may be connected to a human interface such as one or several buttons, keyboards, screen, touch screens, loudspeaker, LED and haptic engine (not shown). The general microprocessor 340 may also be connected preferably to at least one communication interfaces such as a wired interface, such as USB, ethernet, or a proprietary interface, and/or at least one wireless interface, such as NFC, ANT, ZigBee, RF4CE, LoRa, Bluetooth and/or Wi-Fi. Alternatively, the general microprocessor 340 may act as the microcontroller 330 or include the microcontroller 330.

The detection device 325, the general microprocessor 340 and optionally the current generator 310 may be integrated on a printed circuit board (PCB). A power source (not shown) may be connected to all the elements of the sensor device 200, for example a secondary battery, a solar panel and/or a power adaptor.

The sensor device 200 may be integrated as a smart watch, a smart ring, a smart wristband or as a medical device, standalone or connected to a larger medical apparatus. In any case, the sensor device 200 may be included in a case provided with holding means for a user to hold the sensor device on a body part and/or fixing means adapted to fix the sensor device on the body part. The main surface 110 of the substrate 100 with the cavities 180 may be placed in direct contact with the skin or flesh of a patient or user. The pressure applied on the body part be 0.01 N to 0.5 N for example, such as the skin or flesh penetrates the cavities 180.

### Substrate (Second Embodiment)

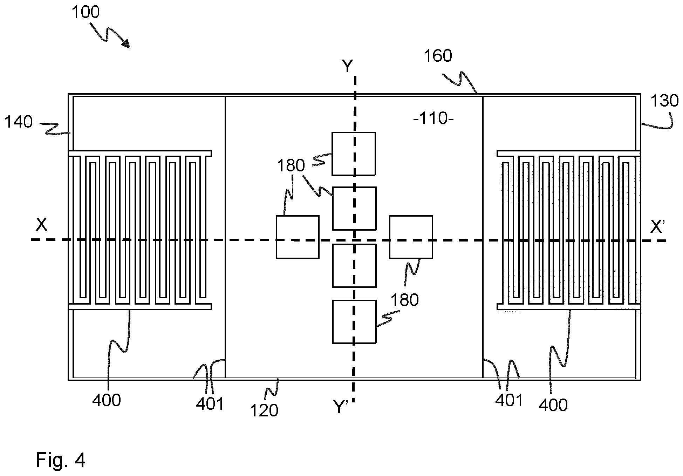

Fig. 4 shows a substrate 100 according to another embodiment of the present disclosure. The same reference numbers as in Figs. 1-3 represents similar technical features. A first difference with the substrate 100 of Fig. 1 is that the substrate 100 of Fig. 4 is provided with six cavities 180, four of them being aligned on a transversal axis Y-Y' of the substrate 100, wherein two cavities 180 are aligned on a longitudinal axis X-X' of the substrate 100. Such a number and arrangement of the cavities 180 may be adapted to a substrate 100 larger and/or more sensitive than that of Fig. 1

A second difference with the substrate 100 of Fig. 1, not linked with the first difference, is the presence of at least one and preferably two or more pairs of interdigitated electrodes 400. A pair of interdigitated electrodes consists of at least two electrode sets that are laid out in a comb-like pattern, with the "fingers" of one electrode set interleaving with those of another, but without touching. Each electrode is made of a conducting material, such as metal, in particular copper or aluminum or alternatively a conductive polymer or ink. The interdigitated electrodes 400 are connected to independent elements of the sensor device for example comprising a microprocessor and/or a microcontroller and a current generator, similarly to what was previously described.

The cavities 180 may be generally located in a central portion of the substate 100, for example covering 20% to 40% of the area of the main surface 110 of Figs. 1 and 4. The interdigitated electrodes 400 of Fig. 4 may be located in side areas of the main surface 110, for example on each lateral side with regard to the portion of the main substrate 110 provided with the cavities 180. In a sensor device (not shown), the interdigitated electrodes 400 may not be in direct contact with the skin or flesh of the patient, but may be covered with insolating sheets or with an casing, materialized by the lines 401 in Fig. 4, the insulating sheets or casing being intended to avoid a direct contact with the skin while letting a small space, for example 0.5 to 4 mm, to let moisture or sweat reaches the interdigitated electrodes 400. Consequently, only the central portion of the main surface 110 may be directly in contact with the skin.

### Measurement Method (ultrasounds)

The sensor device 200 may be adapted to perform the following measurement method of a physiological parameter of an animal or human being. For example, the physiological parameter may be blood sugar, heart rate and/or blood pressure.

Without being bound by any theory, the inventors found that the above physiological parameters may be linked with an ultrasonic wave of a particular frequency. For example, blood sugar may be detected with an ultrasonic wave of 200 kHz and blood density with an ultrasonic wave of 700 kHz. The above substrates and sensor device may be used to detect other physiological parameters and is not limited to the above-recited physiological parameter.

According to the measurement method, a square wave (for example gate shaped) or alternatively a sinusoidal electrical signal of 2 to 10 V of amplitude and with a frequency corresponding to the physiological parameter may be produced by the current generator 310. Each measurement may result from a continuous, alternative and preferably sine wave electrical signal applied between the first electrodes 230, 240 with the specific frequency corresponding to the physiological parameter. The electrical signal may be applied during a short time duration, for example in the tenth of milliseconds.

The ultrasound wave generated by the piezoelectric substrate 100 in response to the electrical signal travels along the longitudinal axis X-X', through the epidermis and the blood vessels of the flesh inserted in the cavities 180 and the substrate 100 captures the wave scattered by the flesh and transforms it back into an electric potential (i.e. a detection signal). The returned detection signal is treated through an ADC (Analog Digital Converter), such as the analogic digital converter 320 before it is read by the microcontroller 330.

The microcontroller 330 or the general microprocessor 340 may then perform a Fast Fourrier Transform on the returned detection signal with a known algorithm such as the Cooley-Tukey algorithm. The signal amplitude at a frequency linked with a specific parameter is then extracted and a value of the physical parameter is obtained through calibration.

Several measurements can be made one after the other in order to obtain the value of several physiological parameters. For example, a first ultrasonic wave may be emitted at a first wavelength and then a second ultrasonic wave may be emitted at a second wavelength. Because the retrieved detection signal is obtained and analysed simultaneously with the emitted wave, the present sensor device can retrieve data and measure several physiological parameters in a short time.

### Calibration (ultrasounds)

A calibration curve provides a direct link between the amplitude of the signal at a particular frequency and the value of the corresponding physical parameter. It is preferably obtained from real-life measurements of a same parameter measured by the sensor device and a medical analysis, for example a blood analysis.

Consequently, a study on 111 sensor devices realized as smart wristbands were evenly distributed between males and females participants (59 males and 52 females), with ages spanning from 21 years old to 66 years old, for the purpose of both calibrating the sensor device and evaluating the sensor device with regard to a third party glucometer. An independent medical laboratory was used to perform this study.

Two measurements from the sensor device were obtained from each participant (at fast and two hours after a meal). Each participant wears the wristband, and an operator takes a screen shot of two values on the screen (i.e. the output in Volts).

At the same time, each participant gets his blood pressure checked by medical staff with a commercial blood pressure monitor. The operator took a picture of the blood pressure displayed. A blood sugar measurement was then performed on each participant with the third-party glucometer and the operator took a picture of the glucometer. Finally, a lab technician took a blood sample from each participant to perform a blood sugar analysis in the independent medical laboratory.

The operator performing the above steps and using the wristband was not aware of the goal of the study and was not affiliated with the inventors. The third-party glucometer was a medical device purchased in a city pharmacy.

Although a proper calibration curve was obtained based on the above study, an additional calibration by machine learning was performed to enhance accuracy with a mathematical model based on the simulation extended to the forth power. Additional parameters were also added, in particular, the age and the gender of each participant, and additional physiological data extracted from the sensor device, in particular the resonance value and the celerity value. The machine learning model was an ensemble voting statistical model. The model was trained over 70% of the datapoints from the above clinical study. Afterwards, the trained model was used on the remaining 30% datapoints.

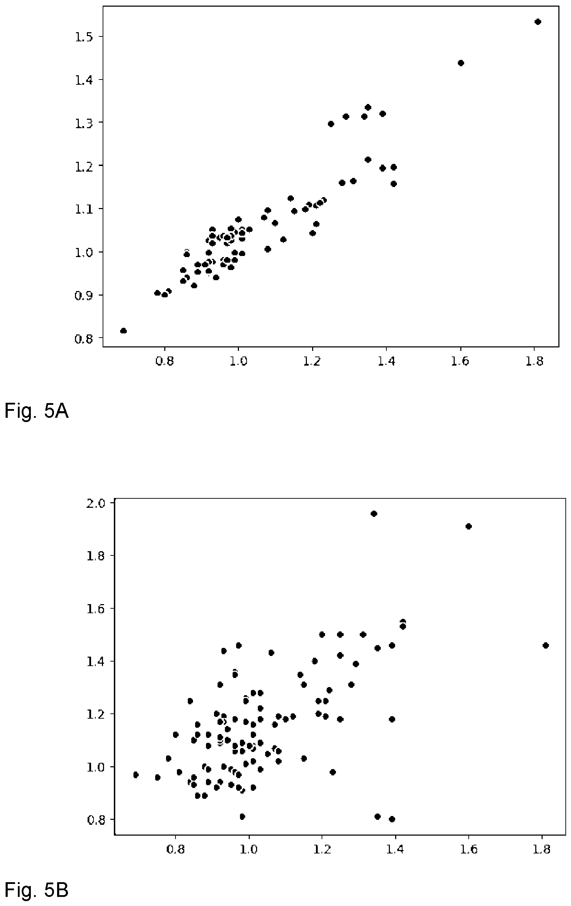

Fig. 5A shows on the Y-axis the value of blood sugar measured by the present sensor device calibrated as described above and on the X-axis the value of blood sugar measured by the medical laboratory, each point representing the values obtained from a single participant.

Fig. 5B shows on the Y-axis the value of blood sugar measured by the third-party glucometer and on the X-axis the value of blood sugar measured by the medical analysis, each point representing the values obtained from a single participant.

In the case of a 100% accurate sensor device, the value acquired by the sensor device should be equal or at least proportional to the value from the medical analysis and all the dots should be aligned on a diagonal line. As visible in Fig. 5A versus Fig. 5B, the accuracy of the present sensor device is greater than the accuracy of the third-party glucometer.

The above study thus allowed to calibrate the present sensor device and to demonstrate an increase in accuracy with regard to a third-party glucometer. The present sensor device may also be realized as a small-size, light and low-cost wristband or watch, in contrast with usual medical devices. Further, measurements made by the present sensor device are fast and only require a limited power consumption.

The same kind of calibration has been performed with a 700 kHz pulse to measure blood density which may be correlated to the systolic blood density and/or blood viscosity, wherein dynamic viscosity and density are linearly related. This means that the present sensor device can perform a measurement of blood density and/or blood viscosity which may lead to a detection of hypertension. Other physiological parameters may also be measured and/or monitored by the present ultrasonic sensor device and corresponding method.

### Measurement Method (Interdigitated Electrodes)

In embodiments having the interdigitated electrodes 400, a change in capacitance or impedance measured by the interdigitated electrodes may be linked with skin moisture, i.e. sweat. Abundant sweating may be linked with a stress level and/or with dehydration and a measurement of this physiological parameter may be used alone or with other physiological parameters to detect a stress level and/or to prevent dehydration, for example for athletes, children, outdoor professional or elderly people.

A factory calibration may be performed to align the electric signal measured with values of sweat, and a second calibration may be performed directly with the patient or user wearing the sensor device including the substrate 100 of Fig. 4. For example, this second calibration may be performed by machine learning for example on the basis of the signal acquired by the interdigitated electrodes, heart rate measurements, declarations of the user, outside temperature, body temperature measurements and/or cortisol measurements.

Although the present disclosure has been described and illustrated in detail, it is clearly understood that the same is by way of illustration and example only and is not to be taken by way of limitations, the scope of the present disclosure being limited only by the terms of the appended claims.

In particular, the present sensor device may be used for nondestructive structural or chemical analysis, for example in the field of food, materials, chemistry and drug, using different ultrasound frequencies. Similarly, the embodiment of Fig. 4 may be used in these different fields, to provide an additional moisture measurement.

## Claims

1. An ultrasonic sensor substrate (100), made of a piezoelectric material and including:
- two opposite main surfaces (110, 120) each having a large surface area,
- two opposite side surfaces (130, 140) each having a small surface area and each adapted to receive a respective first electrode (230, 240),
wherein one of the main surfaces (120) is adapted to receive a second electrode and the other main surface (110) of the main surfaces includes at least two cavities (180).

2. The ultrasonic sensor substrate of claim 1, wherein the two cavities (180) are arranged along a transversal axis (Y-Y') of the ultrasonic sensor substrate (100).

3. The ultrasonic sensor substrate of claim 1 or 2, comprising at least another cavity (180) preferably arranged along a longitudinal axis (X-X') of the ultrasonic sensor substrate (100) with regard to the two cavities (180).

4. The ultrasonic sensor substrate (100) of any of claims 1 to 3, wherein the cavities (180) have a height of 0.5 to 5 mm and a width or diameter of 1 to 5 mm.

5. The ultrasonic sensor substrate (100) of any of claims 1 to 4, wherein the main surfaces (110, 120) and the side surfaces (130, 140) are flat and the side surfaces (130, 140) are orthogonal to at least one of the main surfaces (110, 120).

6. The ultrasonic sensor substrate (100) of any of claims 1 or 4, wherein at least one of the main surfaces (110, 120) is curved and the side surfaces (130, 140) are orthogonal to an extremal tangent of the at least one curved main surface (110, 120).

7. The ultrasonic sensor substrate (100) of any of claims 1 to 6, wherein the piezoelectric material is at least one of PVDF-TrFE, PVDF-graphene and quartz.

8. The ultrasonic sensor substrate (100) of any of claims 1 to 7, wherein the other main surface (110) comprises at least one pair of interdigitated electrodes (400).

9. An ultrasonic sensor device (200) including:
• the ultrasonic sensor substrate (100) of any of claims 1 to 8,
• at least one first electrode (230, 240) on each side surface (130, 140) and
• a second electrode (220) on the one main surface (120),
wherein the second electrode (220) covers at least 50% of the area of the one main surface (120), preferably at least 75% and again preferably at least 90% of the area of the one main surface (120).

10. The ultrasonic sensor device (200) of claim 9, including a detection device (325) connected to one of the first electrodes (230, 240) and to the second electrode (220).

11. The ultrasonic sensor device (200) of any of claims 9 to 10, comprising fixing means adapted to fix the ultrasonic sensor on a body part so that the other main surface (110) applies pressure on the body part.

12. A method to measure a physiological parameter with the ultrasonic sensor device of any of claims 9 to 11, comprising the steps of:
- Generating an electrical signal between the first electrodes (230, 240),
- Detecting a detection signal between one of the first electrodes (230, 240) and the second electrode (220),
- Computing a value of a physiological parameter from the detection signal.

13. The method of claim 12, wherein the electrical signal is continuous and periodic.

14. The method of claim 12 or 13, wherein the electrical signal is alternative.

15. The method of any of claims 12 to 14, wherein the electrical signal has a frequency of more than 1 kHz and less than 1 MHz.
